# EUROPÄISCHE PATENTSCHRIFT

(11) **EP 1 564 558 B1**
(45) Veröffentlichungstag und Bekanntmachung des Hinweises auf die Patenterteilung: **07.12.2005**
(21) Anmeldenummer: 04003295.5
(22) Anmeldetag: 13.02.2004
(51) Int. Cl.: G01N 33/74, G01N 33/68, G01N 33/574

(54) **Verfahren zur Bestimmung der Bildung von Endothelinen zu Zwecken der medizinischen Diagnostik, sowie Antikörper und Kits für die Durchführung eines solchen Verfahrens**
Method for the determination of endothelins for medical diagnostic, antibodies and kits
Procédé pour la détermination d'endothelines pour le diagostic medical, les anticorps et les kits

(43) Veröffentlichungstag der Anmeldung: 17.08.2005
(73) Patentinhaber: B.R.A.H.M.S Aktiengesellschaft, 16761 Hennigsdorf (DE)
(72) Erfinder: Bergmann, Andreas, Dr., 12351 Berlin (DE); Struck, Joachim, Dr., 12161 Berlin (DE)
(74) Vertreter: Andrae, Steffen

(56) Entgegenhaltungen:
- WO-A-00/22439
- HEMSEN A ET AL: "Metabolism of Big endothelin-1 (1-38) and (22-38) in the human circulation in relation to production of endothelin-1 (1-21)" REGULATORY PEPTIDES 1995 NETHERLANDS, Bd. 55, Nr. 3, 1995, Seiten 287-297, XP002284543 ISSN: 0167-0115

## Beschreibung

Die Erfindung betrifft: In vitro-Verfahren zur Bestimmung der Bildung von Endothelinen bei Herz-Kreislauf-Erkrankungen, Entzündungen, Sepsis und Krebs, in Vollblut, Plasma oder Serum eines menschlichen Patienten zu Zwecken der medizinischen Diagnostik, dadurch gekennzeichnet, dass man die Bildung von Endothelin-1 (SEQ ID NO:2) und big-Endothelin-1 (SEQ ID NO:3) dadurch bestimmt, dass man solche C-terminalen Fragmente des Präpro-Endothelins-1 (SEQ ID NO:1) bestimmt, die von Antikörpern erkannt werden, die an Peptide binden, die Peptidsequenzen im Bereich der Aminosäuren 93 bis 212 des Präpro-Endothelins-1 entsprechen.

Wenn im Rahmen der vorliegenden Anmeldung einfach von "Endothelin" gesprochen wird, steht dieser Begriff in erster Linie für Endothelin-1 (ET-1). Eine entsprechende Aussage ist jedoch häufig auch für andere Isoformen von Endothelinen gültig, weshalb eine Beschränkung auf Endothelin-1 häufig nicht erforderlich erscheint und sich die Erfindung in einem weiteren Sinne auch auf andere Endotheline erstrecken soll.

In dieser Beschreibung wird dabei der Begriff "Diagnostik" grundsätzlich als vereinfachender Oberbegriff verwendet, der insbesondere auch Prognostik/Frühprognostik und therapiebegleitende Verlaufskontrolle einschließen soll.

Die Bestimmungen erfolgen insbesondere mittels spezifischer immundiagnostischer Verfahren, insbesondere mittels Immunoassays eines Typs, bei dem mit mindestens einem markierten Antikörper gearbeitet wird (Sandwichassay; kompetitiver Assay, z.B. nach dem SPALT oder SPART-Prinzip).

Endothelin-1 (ET-1), ein 21 Aminosäuren umfassendes Peptid, ist der stärkste bekannte Vasokonstriktor. Seit seiner Entdeckung im Jahr 1988 durch Yanagisawa und Kollegen [27; Zahlenangaben in eckigen Klammern beziehen sich auf die beigefügte Literaturliste] sind Biosynthese, Wirkungsweise und Assoziation mit Krankheiten umfangreich untersucht worden und in aktuellen Review-Artikeln zusammengefaßt [1, 7, 12, 17, 24]. Es existieren drei, von unterschiedlichen Genen kodierte Isoformen von Endothelin (Endothelin-1, Endothelin-2, Endothelin-3), wovon Endothelin-1 in den größten Konzentrationen vorliegt und am wirksamsten ist. Endothelin-1 wird in Endothelzellen, in der Lunge, im Herz, in der Niere und im Gehirn synthetisiert. Das primäre Translationsprodukt des humanen Endothelin-1 Gens ist ein 212 Aminosäuren umfassendes Peptid, Präpro-Endothelin-1 (SEQ ID NO:1). Im Sekretionsprozess wird durch die Signalpeptidase eine kurze N-terminale Signalsequenz (Aminosäuren 1-17) des Präpro-Endothelins entfernt. Das dabei erhaltene Pro-Endothelin wird anschließend durch die Protease Furin an dibasischen Aminosäurepaaren zu einem biologisch inaktiven, 38 Aminosäuren umfassenden Peptid big-Endothelin (SEQ ID NO:3) prozessiert, woraus schließlich mittels Endothelin-coverting enzymes (ECEs) das reife, biologisch wirksame Endothelin-1 (SEQ ID NO:2) gebildet wird. Endothelin wirkt über die Bindung an spezifische Rezeptoren, die auf Muskelzellen, Myozyten und Fibroblasten lokalisiert sind. Diese Bindung führt zum Efflux von Calcium, Aktivierung von Phospholipase C und Inhibierung der Na/K ATPase. Neben der vasokonstriktiven Wirkung hat Endothelin auch wachstumsregulierende Eigenschaften.

Angesichts der nachweisbaren und zu vermutenden zahlreichen und gravierenden physiologischen Wirkungen von Endothelinen, insbesondere Endothelin-1, wurden seit dem Zeitpunkt seiner Identifizierung verschiedene Assays zu seiner immundiagnostischen Bestimmung entwickelt und zu Messungen von Endothelin(en) insbesondere in humanen Plasmen eingesetzt. Die Ergebnisse derartiger Bestimmungen sind Gegenstand zahlreicher Veröffentlichungen.

Erhöhte Plasmakonzentrationen von Endothelin-1 und big-Endothelin sind für verschiedene Krankheitsbilder beschrieben worden [17]. Dazu zählen kardiovaskuläre Erkrankungen [1] (u.a. Pulmonary Hypertension [21], Atherosclerosis [13], Congestive heart failure [25], Herzinfarkt [20]), Sepsis und septischer Schock [11, 22, 23], Krebs [2, 3, 15, 18], u.a..

Die zu den Messungen von Endothelinen in Plasmaproben verwendeten Immunoassays (vgl. die Übersicht in [17]) gehörten insbesondere zum Typ der Radioimmunoassays (mit markiertem Endothelin-1 als Kompetitor) oder zum EIA/ELISA-Typ und zielten ausschließlich auf die Bestimmung des Endothelins ab bzw. auf die Bestimmung einer Endothelin-Immunreaktivität. Assays vom RIA-Typ weisen dabei eine geringere Spezifität auf und erfassen auch die Endothelin-Sequenz enthaltende verwandte Peptide.

Es wurde jedoch festgestellt, dass Endothelin-1 (ET-1) eine extrem kurze Verweildauer in der Zirkulation aufweist und dass es bereits nach 1-2 min aus dem Kreislauf entfernt wird [6]. Da Endothelin-1 in Blut und Plasma als stabil gilt [6], wird als wichtigster Grund für die kurze Verweildauer seine Verteilung in andere Gewebe und seine schnelle und hochaffine Bindung an Rezeptoren angesehen. In bestimmten Geweben und Körperflüssigkeiten konnten folglich deutlich höhere Endothelin-1-Konzentrationen ermittelt werden als z.B. im Plasma [1, 7]. Die Validität der Bestimmung von ET-1 in Plasmaproben wurde angesichts dieser Umstände in ernste Zweifel gezogen [17]. Es ist nämlich anzunehmen, dass für die physiologischen Wirkungen des Endothelins (ET-1) nicht die in einer Plasmaprobe ermittelbaren momentanen, u.U. nur einen Durchgangszustand widerspiegelnden ET-1-Konzentrationen wichtig sind, sondern dass die Summe aller im Organismus vorhandenen freien und gebundenen, z.B. gewebe- und rezeptorgebundenen physiologischen ET-1-Konzentrationen von viel größerer Relevanz sind.

Die Bestimmung des ET-1-Vorläufers, des sogenannten big-Endothelins ("big ET-1"; SEQ ID NO:3) weist gegenüber der Bestimmung von ET-1 den Vorteil auf, dass die Verweildauer von "big-ET-1" in der Zirkulation deutlich höher ist als die des daraus freigesetzten ET-1. In einer Reihe von Untersuchungen wurde daher anstelle des eigentlichen Endothelins dieses "big-Endothelin" bestimmt. Zu seiner spezifischen Bestimmung wurden insbesondere Assays vom Sandwichtyp verwendet, die eine sichere Unterscheidung des big-Endothelins-1 vom prozessierten ET-1 und anderen Endothelinen ermöglichen [4, 8, 10]. Sie zeigten, dass bei bestimmten Erkrankungen die erhöht gemessenen ET-Immunreaktivitäten auf big-ET zurückgeführt werden können.

Die selektive Messung des big-ET-1 stellt nur eine graduelle Verbesserung, jedoch keine tatsächliche Lösung des Problems dar, denn auch big-Endothelin kann in der Blutzirkulation schnell zu Endothelin prozessiert werden [1, 5, 9]. Es hat damit ebenfalls eine relativ kurze biologische Halbwertszeit (20-25 Minuten) [10], und folglich repräsentiert ein Messwert für das im Plasma bestimmbares big-Endothelin ebenfalls nur eine momentane Plasmakonzentration und spiegelt nicht die tatsächlich physiologisch wirksamen Konzentrationen an Endothelin wider. Unter den Bedingungen einer Erkrankung physiologisch gebildetes, jedoch bereits prozessiertes und in Geweben bzw. an Rezeptoren gebundenes ET-1 wird bei der Bestimmung von big-ET-1 in Plasma nicht erfaßt. Die Gesamtmenge an physiologisch aktivem Endothelin wird daher auch bei einer Messung von big-Endothelin unterschätzt. Der Versuch einer ergänzenden spezifischen Messung des bei der enzymatischen Spaltung des big-ET-1 neben ET-1 gebildeten C-terminalen Peptidfragments des big-ET-1 (mit den Aminosäuren 74-90 des Präpro-Endothelins bzw. den Aminosäuren 22-38 des big-Endothelins) ergab, dass dieses Peptid noch weniger stabil ist als ET-1 und sich daher für Messungen nicht eignet [10].

Für die Bemessung von Bereichen des Pro-Endothelin außerhalb des big-Endothelin ist aus dem Stand der Technik lediglich ein kommerzieller kompetitiver Test bekannt (N-terminaler Bereich 18-50, kommerziell erhältlich bei Phoenix Pharmaceuticals; Anwendung für die Sepsisdiagnostik beschrieben in WO00/22439). Zur Stabilität und Natur des mit diesem Assay zu bemessenden Analyten sind keine Informationen veröffentlicht.

Die vorliegende Erfindung hat es sich zur Aufgabe gemacht, ein Bestimmungsverfahren zu entwickeln, das die endogene Bildung von big-Endothelin und Endothelin, d.h. die gesamte physiologische Konzentration und damit Wirkung des Endothelins, verläßlicher widerspiegelt als die bisherigen Bestimmungen von ET bzw. big-ET in Plasma.

Ein solches Verfahren soll valide und routinetauglich sein und zuverlässige Werte für die physiologische Produktion von ET (ET-1) und/oder seinen Vorläufern bei verschiedenen Krankheitszuständen, insbesondere bei Sepsis oder anderen Krankheitszuständen, bei denen erhöhte Werte für Endothelin eine Rolle spielen, liefern können.

Diese Aufgabe wird erfindungsgemäß dadurch gelöst, dass zu Diagnosezwecken in einer Vollblut-, Plasma- oder Serumprobe eines humanen Patienten nicht ET oder big-ET bestimmt wird, sondern ein vergleichsweise langlebiges Präpro- bzw. Pro-Endothelin-Teilpeptid, das die ET- bzw. big-ET-Sequenzen nicht enthält, insbesondere ein C-terminales Teilpeptid, das die Aminosäuren 168-212 des pre-proET-1 wenigstens enthält.

Anspruch 1 betrifft die Lehre der vorliegenden Erfindung. Vorteilhafte und derzeit bevorzugte Ausgestaltungen der Erfindung sind den Unteransprüchen zu entnehmen.

Die Erfindung beruht auf experimentellen Untersuchungen der Anmelderin, in denen sie zeigen konnte, dass solche Teile des Präpro-Endothelins, die keine direkten Vorläufer des Endothelins darstellen, für Messzwecke geeignete langlebige Peptide umfassen, die in Blutproben zuverlässig und mit einer hohen klinischen Wertigkeit gemessen werden können.

Endothelin-1 wird physiologisch durch Prozessierung des größeren Vorläufermoleküls Präpro-Endothelin (SEQ ID NO:1) bzw. des daraus erhaltenen sekretierten Pro-Endothelins gebildet. Bei einer solchen Prozessierung müssen neben big--Endothelin (und daraus Endothelin) in primär stöchiometrischen Mengen weitere Peptide entstehen, die bisher jedoch noch nie Gegenstand wissenschaftlicher Untersuchungen waren und über deren mögliche weitere Prozessierung und Stabilität nichts bekannt geworden ist. Am Beginn der Untersuchungen der Anmelderin stand die Hoffnung, dass sich zeigen könnte, dass sich wenigstens eines der hypothetischen weiteren Peptid-Spaltprodukte in Blutproben (Vollblut-,Plasma- oder Serumproben) finden und als relativ stabil erweisen würde und das daher geeignet sein könnte, als Maß für die physiologische Bildung von Endothelinen unabhängig von einer aktuellen in Plasma messbaren Endothelin-Konzentration zu dienen.

Die Bemessung eines solchen Spaltprodukts könnte somit die gesuchte Methode zur Bestimmung der physiologischen Endothelin-Konzentration bzw. -Produktion darstellen, die in den Ansprüchen als Bestimmung der "Bildung von Endothelinen" bezeichnet wird. Mit diesem Begriff wird darauf verwiesen, dass - unter der Annahme nur eines, nämlich des einzigen bekannten Bildungswegs von Endothelin-1 aus Präpro-Endothelin - die im Zusammenhang mit einer Erkrankung gebildeten physiologischen Konzentrationen des Endothelins-1 nur der Menge des vorher prozessierten Präpro-Endothelins bzw. Pro-Endothelins entsprechen können. Stellen die neben big-Endothelin bzw. Endothelin in gleicher stöchiometrischer Konzentration gebildeten Teilpeptide stabile "Stoffwechselschlakken" dar, die weder an Rezeptoren gebunden werden noch in Geweben verteilt werden, müssen sie sich in der Zirkulation wiederfinden. Ohne damit zwingend einen bestimmten physiologsichen Mechanismus implizieren zu wollen, kann daher die "Bestimmung/Messung der Bildung von Endothelinen" auch als Messung "der sekretorischen Aktivität" bzw. der "sekretorischen Pro-Endothelin-Produktion" angesehen werden.

In dieser Anmeldung werden die zu bestimmenden Peptidfragmente als "langlebig" gekennzeichnet. Mit diesem Begriff ist gemeint, dass die Verweildauer der zu bestimmenden Peptidfragment in der Zirkulation (im Vollblut) erheblich länger ist als die des Endothelins bzw. der big-Endothelin-Fragmente. Insbesondere ist mit "langlebig" gemeint, dass solche Peptidfragmente in Vollblut und einem daraus gewonnenen Plasma keiner weiteren raschen proteolytischen Spaltung unterliegen und, verglichen mit der Geschwindigkeit der Bindung des Endothelins an Rezeptoren und der proteolytischen Spaltung spaltbarer Fragmente, mit einer deutlich langsameren Geschwindigkeit aus der Zirkulation bzw. dem Stoffwechsel entfernt werden.

Aufgrund der genannten längeren Stabilität oder "Langlebigkiet" ist in der Anwesenheit derartiger Fragmente die Information über die bereits abgelaufenen sekretorischen Aktivitäten für einen Zeitraum gespeichert, der wenigstens für eine unproblematische Messung geeignet ist. Nimmt man z.B. an, dass die Endothelin-Vorläufer im Sinne einer einzigen kurzzeitigen Ausschüttung freigesetzt werden, entspricht die nach einer gewissen Zeit messbare Menge an "langlebigen" Fragmenten der ursprünglich ausgeschütteten Menge, vermindert nur um eine Menge, die mit der physiologischen Halbswertzeit des zu messenden Peptidfragments in der Zirkulation verknüpft ist. Nimmt man andererseits z.B. eine mehr oder weniger kontinuierliche Produktion des Endothelin-Vorläufers während des Krankheitsgeschehens an, spiegelt sich in der messbaren Konzentration eines im obigen Sinne langlebigen Peptidfragments die zurückliegende physiologische Produktion des Vorläufers kumulativ wieder, wiederum nur vermindert um die im gleichen Zeitraum erfolgte Konzentrationsverminderung des Peptidfragments nach Maßgabe seiner physiologischen Clearance-Geschwindigkeit. Das aktive Endothelin, oder sein Vorläufer big-Endothelin, können im gleichen Zeitraum längst prozessiert bzw. aus der Zirkulation entfernt und z.B. an Rezeptoren gebunden und damit nicht mehr messbar sein. Je langlebiger ein Peptidfragment ist, bzw. je geringer seine Clearance-Geschwindigkeit ist, desto geringer ist der Einfluss des Messzeitpunkts auf die Korrektheit der Bestimmung der o.g. "Bildung" eines Biomarkers, d.h. des Endothelins. Eine über längere Zeiträume konstante Konzentration bedeutet in diesem Bild, dass sich Bildung und Clearance die Waage halten. Sinkt die Konzentration, so kann das darauf hindeuten, dass die Sekretion des Vorläufermoleküls (z.B. des Proendothelins) aufgehört hat, z.B. weil die molekularen Speicher erchöpft sind, und die zu beobachtenden Konzentrationsveränderungen nur noch durch die Clearance-Geschwinsdigkeit bestimmt werden.

Die Ergebnisse der Messung eines langlebigen Peptidfragments ohne bekannte physiologische Funktion liefert somit sowohl quantitativ als auch qualitativ andere Ergebnisse als eine Messung eines eher kurzlebigen aktiven Peptids oder seines ebenfalls relativ kurzlebigen Vorläufers.

Die nachfolgend näher beschriebenen Untersuchungen der Anmelderin zeigten, dass der oben beschrieben Ansatz im Falle der Bestimmung der Bildung von Endothelinen fruchtbare Ergebnisse liefert.

Die durchgeführten Untersuchungen und die signifikantesten Ergebnisse dieser Untersuchungen werden nachfolgend genauer erläutert, wobei auf Figuren Bezug genommen wird.

Dabei zeigen:
- Figur 1: eine typische Standardkurve für den im experimentellen Teil genauer beschriebenen derzeit bevorzugten Sandwichassay mit zwei Antikörpern, die an Aminosäuresequenzen binden, die den Positionen 168-181 und 200-212 des Präpro-Endothelins-1 entsprechen, zur Bestimmung einer C-terminalen Pro-Endothelin-Peptidsequenz in humanem Plasma;
- Figur 2: ein Diagramm, das zeigt, dass bei der Lagerung von EDTA-Plasmaproben von septischen und kardiologischen Patienten bei Raumtemperatur über 12 Stunden kein nennenswerter Verlust der Immunreaktivität in einem Assay gemäß Figur 1 eintritt;
- Figur 3a: die Messung von Plasmen von 5 Gruppen von menschlichen Patienten mit verschiedenen Erkrankungen/-Diagnosen, gegenübergestellt den Messungen für augenscheinlich Gesunde; die punktierte Linie zeigt den maximalen bei Gesunden gefundenen Wert an (Linie für 100% Spezifität, bezogen auf gesunde Kontrollen);
- Figur 3b: eine Figur 3a entsprechende Darstellung, für vier weitere Gruppen von Patientenplasmen;

Das erfindungsgemäße Verfahren betrifft in seinem allgemeinsten Aspekt die Bestimmung eines relativ langlebigen Peptidfragments des Pro-Endothelins-1, das die Aminoäuresequenzen des Endothelins-1 bzw. seines Vorläufers big-Endothelin nicht enthält, in Vollblut-, Plasma- bzw. Serumproben, d.h. in der Zirkulation von Patienten, zur indirekten Bestimmung der Bildung von Endothelinen, insbesonder von Endothelin-1, bei schweren Erkrankungen. Gemäß einer bevorzugten Ausführungsform ist das bestimmte Peptidfragment ein C-terminales Fragment, an das zwei Antikörper binden, die an Peptide mit Aminosäuresequenzen binden, die den Positionen 168-181 und 200-212 des Präpro-Endothelins-1 entsprechen.

Für die praktische Umsetzung der Erfindung sind besonders bevorzugt nicht-kompetitive Sandwichassays, z.B. der Art, wie er für die weitergehenden vertiefenden Untersuchungen verwendet wurde und nachfolgend genauer beschrieben wird, vorgesehen.

Nicht-kompetitive Sandwich-Immunoassays (Zweiseiten-Immunoassays) haben gegenüber kompetitiven Immunoassays eine Reihe von Vorteilen, zu denen gehört, dass sie besser als Festphasenassays (heterogene Assays) ausgelegt werden können, in der Handhabbarkeit robuster sein können, Meßergebnisse mit einer höheren Sensititivät liefern können und sich auch besser für eine Automatisierung und Serienmessung eignen. Außerdem können sie im Vergleich mit kompetitiven Immunoassays, die mit nur einem Typ von Antikörper arbeiten, auch zusätzliche Aussagen liefern, indem Sandwich-Immunoassays nur solche Moleküle bzw. Peptide erkennen, bei denen beide Bindungsstellen für die für die Sandwichbildung verwendeten Antikörper auf dem gleichen Molekül vorhanden sind.

Die verwendbaren Antikörper können grundsätzlich beliebige geeignete monoklonale und/oder polyklonale Antikörper sein, wobei jedoch derzeit affinätsgereinigte polyklonale Antikörper bevorzugt werden.

Besonders bevorzugt werden die Antikörper durch Immunisierung eines Tiers, insbesondere Schafs, mit einem Antigen erhalten, das eine synthetische Peptidsequenz enthält, die einer kurzen Aminosäurensequenz des Präpro-Endothelins-1 entspricht und einen zusätzlichen Cysteinrest am N-Terminus aufweist. Im nachfolgenden experimentellen Teil werden insbesondere Antikörper bzw. deren Verwendung in einem Assay beschrieben, die an die Aminosäuresequenzen 161-181 und 200-212 binden. Es wurden jedoch im Rahmen der Untersuchungen auch zusätzliche Antikörper eingesetzt, die entsprechend an die Positionen 184-203 und 136-148 binden. Die mit diesen weiteren Antikörpern bei Messungen erhaltenen ergänzenden Ergebnisse werden in dieser Anmeldung nur pauschal diskutiert.

Bei einer bevorzugten Ausführungsform wird das Verfahren als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests zur Bestimmung in Vollblutproben verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Es liegt somit im Rahmen der vorliegenden Erfindung, das erfindungsgemäße Verfahren auch als Schnelltest auszugestalten.

Das erfindungsgemäße Verfahren kann ferner als homogenes Verfahren ausgestaltet werden, bei dem die aus den beiden Antikörpern und dem nachzuweisenden Peptidfragment gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE® (Time Resolved Amplified Cryptate Emission) bzw. KRYPTOR® angebotene Technologie zu verweisen, die die Lehren der o.g. Anmeldungen umsetzt.

Es zeigte sich bei den Untersuchungen der Anmelderin, dass die erfindungsgemäße Bestimmung des C-terminalen Peptidfragments des Präpro-Endothelins-1 hochinteressante und relevante Meßergebnisse liefert. Diese Aussage gilt, wie nachfolgend gezeigt wird, nicht nur für die Sepsisdiagnostik, sondern auch für die Kardialdiagnostik und die Krebsdiagnostik.

Es wird ferner davon ausgegangen, dass das erfindungsgemäße Bestimmungsverfahren besonders vorteilhaft auch im Rahmen einer sogenannten Multiparameterdiagnostik durchgeführt werden kann, und zwar sowohl auf dem Gebiet der Kardialdiagnostik als auch der Sepsis- und der Krebsdiagnostik. Weitere dabei bestimmte Parameter sind beispielsweise die Kardialparameter ANP, BNP, proANP, proADM oder proBNP oder Sepsisparameter, die z.B. aus der Gruppe ausgewählt sind, die besteht aus Anti-Gangliosid-Antikörpern, den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (sCY1F), den Peptiden Inflammin und CHP, anderen Peptid-Prohormonen, der Glycin-N-Acyltransferase (GNAT), der Carbamoylphosphat Synthetase 1 (CPS 1) und dem C-reaktiven Protein (CRP) oder Fragmenten davon. Bei den genannten Multiparameter-Bestimmungen ist vorgesehen, die Messergebnisse für mehrere Parameter gleichzeitig oder parallel zu bestimmen und z.B. mit Hilfe eines Computerprogramms, das auch diagnostisch signifikante Parameterkorrelationen nutzt, auszuwerten.

Nachfolgend wird die Erfindung durch eine Beschreibung der Herstellung der bevorzugten Assay-Komponenten, der Durchführung einer bevorzugten Ausführungsform eines Assays vom Sandwich-Typ und der unter Verwendung eines solchen Assays erhaltenen Ergebnisse der Bestimmung eines C-termianlen Peptidfragments in EDTA-Plasmen von Kontrollpersonen, und von Sepsis-, Herz- und Krebspatienten näher erläutert.

### Experimenteller Teil

### A. Materialien und Methoden

### 1. Peptidsynthesen

Abgeleitet von der bekannten Aminosäuresequenz des humanen Präpro-Endothelin-1 (SEQ ID NO:1) wurden drei Bereiche ausgewählt (Pos. 168-181, 184-203, 200-212). Jeweils ergänzt um einen N-terminalen Cystein-Rest wurden diese Bereiche nach Standardverfahren als lösliche Peptide chemisch synthetisiert, gereinigt, mittels Massenspektrometrie und Reversed Phase HPLC qualitätskontrolliert und in Aliquots lyophilisiert (Firma JERINI AG, Berlin, Deutschland). Die Aminosäuresequenzen der Peptide lauten:
Peptid PCT15 (168-181 + N-terminales Cystein)
Peptid PCW14 (200-212, + N-terminales Cystein)
Peptid PNR20 (184-203; + N-terminales Cystein)

Desweiteren wurde als Standard zur Eichung der Assays folgendes Peptid synthetisiert:
Standard Peptid PSW44 (169-212)

### 2. Konjugation und Immunisierung

Mittels MBS (m-Maleimidobenzoyl-N-hydroxysuccinimid Ester) wurden die Peptide PCT15 und PCW14 an das Trägerprotein KLH (Keyhole limpet hemocyanin) konjugiert (s. Arbeitsanleitung "NHS-Esters-Maleimide Crosslinkers", Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### 3. Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert.

Dazu wurden zunächst die Peptide PCT15 und PCW14 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen beide Peptide wurde wie folgt durchgeführt:

Die Peptid-Säulen wurden zunächst drei mal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt . Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen à 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach Proteinbestimmung der Pools mittels der BCA-Methode ergaben sich Ausbeuten von 97 mg für den anti-PCT15 Antikörper 0407-pAk und 60 mg für den anti-PCW14 0410-pAk Antikörper.

### 4. Markierung

Der anti-PCW14 0410-pAk Antikörper wurde wie folgt behandelt:

Über eine NAP-5 Gelfiltrationssäule (Pharmacia) wurden 500 µl des gereinigten Antikörpers in 1 ml 100 mM Kalium-Phosphatpuffer (pH 8,0) nach Arbeitsanleitung umgepuffert. Die Proteinkonzentationsbestimmung der Antikörperlösung ergab einen Wert von 1,5 mg/ml.

Zur Chemilumineszenzmarkierung des Antikörpers wurden 67 µl der Antikörperlösung mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsanastz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorptionsverhältnis 368 nm/280 nm als Maß für den Markierungsgrad des Antikörpers betrug am Peak 0.10. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### 5. Kopplung

Der anti-PCT15 Antikörper 0407-pAk wurde wie folgt behandelt:

Bestrahlte 5 ml Polystyrolröhrchen (Firma Greiner) wurden mit gereinigtem Antikörper wie folgt beschichtet: Der Antikörper wurde in 50 mM Tris, 100 mM NaCl, pH 7,8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### B. Durchführung und Auswertung des Immunoassays

Es wurde ein Assaypuffer folgender Zusammensetzung hergestellt: 100 mM Natriumphosphat, 150 mM NaCl, 5% Bovines Serum Albumin (BSA), 0.1% unspezifische Schaf IgG, 0.1% Natriumazid, pH 7.4

Als Standardmaterial diente das o.g. chemisch synthetisierte Peptid (Peptid PSW44), das den Positionen 169-212 von Präpro-Endothelin-1 entspricht. Dieses wurde seriell in Pferdenormalserum (Firma SIGMA) verdünnt. Den so hergestellten Standards wurden Konzentrationen gemäß der Einwaage an Peptid zugeschrieben.

Meßproben waren EDTA-Plasmen von augenscheinlich Gesunden, von Patienten mit Sepsis und von Patienten mit verschiedenen Herz-Kreislauf-Erkrankungen.

In die Teströhrchen wurden 50 µl Standards bzw. Proben sowie 200 µl Assaypuffer pipettiert. Es wurde zwei Stunden bei 22°C unter Schütteln inkubiert. Dann wurde 4 x mit je 1 ml Waschlösung (0.1% Tween 20) pro Röhrchen gewaschen und abtropfen gelassen. Dann wurden 200 µl Assaypuffer, enthaltend 1 Million RLU (relative light units) des MA70-markierten Antikörpers, pipettiert. Es wurde zwei Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurden die Konzentrationen der Proben an der Standardkurve abgelesen.

### C. Ergebnisse

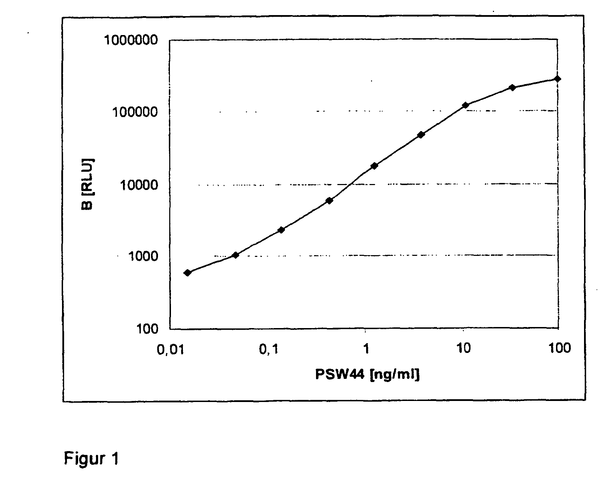

Der mit dem entwickelten Sandwichimmunoassay (Antikörper gegen die Positionen 168-181 und 200-212) bemessbare Analyt wird im Folgenden als C-terminales-Pro-Endothelin oder Ct-Pro-Endothelin bezeichnet. Eine typische Standardkurve für den entwickelten Test ist in Figur 1 gezeigt. Mit dem Test ließen sich noch Ct-Pro-Endothelin-Konzentrationen von deutlich unterhalb 50 pg/ml bestimmen.

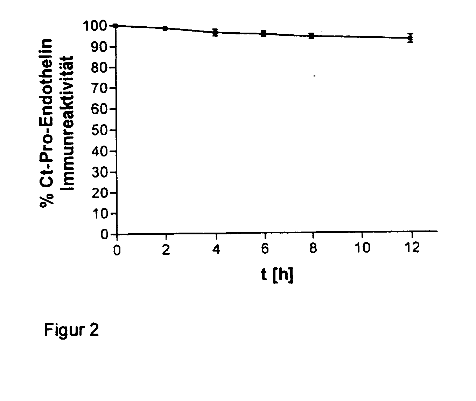

Zur Prüfung der Frage, ob bei einer Messung des C-terminalen Peptidfragments mit Problemen aufgrund einer unzureichenden Stabilität in einer Probe bzw. Messlösung gerechnet werden muß, wurden 5 Sepsis-Plasmen jeweils frisch und nach einer 12 stündige Lagerung bei Raumtemperatur vermessen. Die Ergebnisse sind in Figur 2 zusammengefaßt. Sie zeigen, dass nach der 12tägigen Lagerung die Immunreaktivität mit noch ca. 93% der anfänglich gemessenen Immunreaktivität nahezu unverändert war. Diese nachgewiesene Stabilität ist unter Handhabungsgesichtspunkten für die Diagnostik ein großer Vorteil.

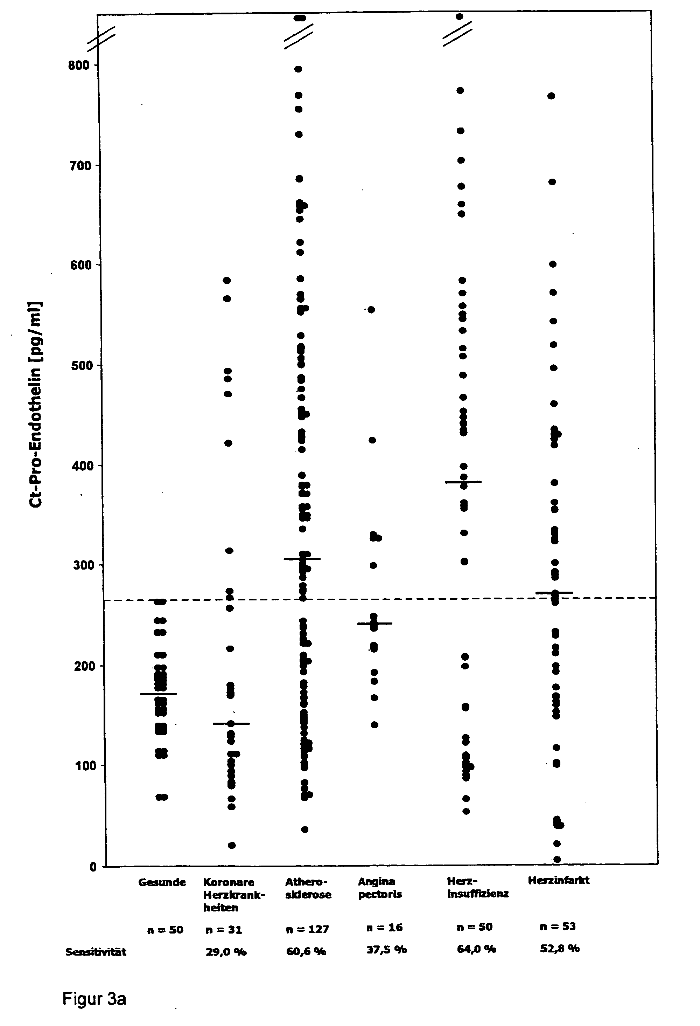

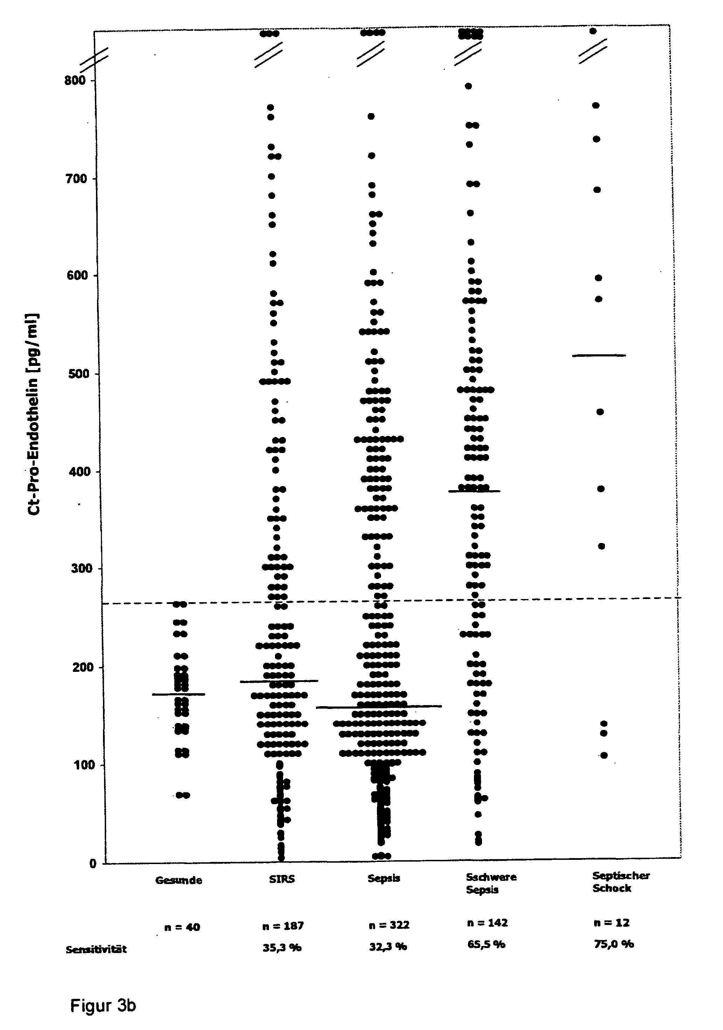

Mit dem Test wurden Plasmen von kardiologischen sowie septischen Patienten vermessen. Die erhaltenen Ergebnisse sind in den Figuren 3a und 3b gezeigt. Für alle untersuchten kardiologischen Krankheitsbilder wurden gegenüber normalen Kontrollen erhöhte Werte gefunden. Ebenfalls erhöhte Werte fanden sich für Patienten mit SIRS (systemic inflammatory response syndrome) und septische Bedingungen. Die diagnostische Sensitivität (bei gegebener 100% Spezifität bezogen auf gesunde Kontrollen) erhöhte sich dabei mit dem Schweregrad der Erkrankung: Sepsis 32,3%, schwere Sepsis 65,5%, und septischer Schock 75%.

Wurden die Proben mit einem modifizierten Assay gemessen, bei dem einer der Antikörper des o.g. Sandwichassays durch einen Antikörper ersetzt war, der die Aminosäuren 184-203 des Präpro-Endothelins-1 erkannte, wurden erwartungsgemäß im wesentlichen identische Ergebnisse erhalten.

Dagegen konnten dann, wenn einer der verwendeten Antikörper eine Aminosäuresequenz erkannte, die näher in Richtung des N-Terminus des Präpro-Endothelins lokalisiert ist (32-52 oder 136-148), keine gegenüber Gesunden erhöhte Messwerte erhalten werden. Das deutet darauf hin, dass Pro-Endothelin als solches nicht in den vermessenen Plasmaproben vorhanden war und nicht nur unter Bildung von big-Endothelin proteolytisch prozessiert wird, sondern dass auch die dabei freigesetzet C-terminale Sequenz 93-212 noch weiter gespalten wird, wobei mindestens eine solche Spaltstelle im Bereich der Aminosäuren 149-167 liegen muss. Diese Aussage gilt für die Plasmen von Patienten mit den untersuchten Erkrankungen. Es ist jedoch nicht auszuschließen, dass bei anderen Patientengruppen z.B. das gesamte C-terminale Fragment 93-212 erhalten bleibt und seine selektive Messung diagnostisch relevante Ergebnisse liefern kann.

### Literaturliste

1. Agapitov AV, Haynes WG. Role of endothelin in cardiovascular disease. J Renin Angiotensin Aldosterone Syst 2002;3:1-15
2. Arun C, Swift B, Porter KE, West KP, London NJ, Hemingway DM. The role of big endothelin-1 in colorectal cancer. Int J Biol Markers 2002;17:268-74
3. Asham EH, Loizidou M, Taylor I. Endothelin-1 and tumour development. Eur J Surg Oncol 1998;24:57-60
4. Aubin P, Le Brun G, Moldovan F, Vilette JM, Créminon C, Dumas J, Homyrda L, Soliman H, Azizi M, and Fiet J, Sandwich-type enzyme immunoassay for big endothelin-1 in plasma: concentrations in healthy human subjects unaffected by sex or posture, Clin Chem 43:1, 64-70 (1997)
5. Corder R, Vane JR. Radioimmunoassay evidence that the pressor effect of big endothelin-1 is due to local conversion to endothelin-1. Biochem Pharmacol 1995;49:375-80
6. de Nucci G, Thomas R, D'Orleans-Juste P, Antunes E, Walder C, Warner TD, Vane JR. Pressor effects of circulating endothelin are limited by its removal in the pulmonary circulation and by the release of prostacyclin and endothelium-derived relaxing factor. Proc Natl Acad Sci U S A 1988;85:9797-800
7. Goraca A. New views on the role of endothelin (minireview). Endocr Regul 2002;36:161-7
8. Haug C, Koenig W, Hoeher M, Kochs M, Hombach V, Gruenert A, and Osterhues H, Direct enzyme immunometric measurement of plasma big endothelin-1 concentrations and correlation with indicators of left ventricular function. Clin Chem 44:2 239-243 (1998)
9. Haynes WG, Webb DJ. Contribution of endogenous generation of endothelin-1 to basal vascular tone. Lancet 1994;344:852-4
10. Hemsen A, Ahlborg G, Ottosson-Seeberger A, Lundberg JM. Metabolism of Big endothelin-1 (1-38) and (22-38) in the human circulation in relation to production of endothelin-1 (1-21). Regul Pept 1995;55:287-97
11. Hirata Y, Mitaka C, Emori T, Amaha K, Marumo F. Plasma endothelins in sepsis syndrome. Jama 1993;270:2182
12. Iskit AB, Guc O. Effects of endothelin and nitric oxide on organ injury, mesenteric ischemia, and survival in experimental models of septic shock. Acta Pharmacol Sin 2003;24:953-7
13. Lerman A, Edwards BS, Hallett JW, Heublein DM, Sandberg SM, Burnett JC, Jr. Circulating and tissue endothelin immunoreactivity in advanced atherosclerosis. N Engl J Med 1991;325:997-1001
14. Mathew V, Lerman A. Clinical implications of a sandwich enzyme immunoassay for big endothelin-1. Clin Chem 1997;43:9-10
15. Nelson JB, Hedican SP, George DJ, Reddi AH, Piantadosi S, Eisenberger MA, Simons JW. Identification of endothelin-1 in the pathophysiology of metastatic adenocarcinoma of the prostate. Nat Med 1995;1:944-9
16. Pittet JF, Morel DR, Hemsen A, Gunning K, Lacroix JS, Suter PM, and Lundberg JM, Elevated Plasma Endothelin-1 Concentrations Are Associated with the Severity of Illness in Patients with Sepsis. Ann. Surg., Vol. 213, No.3, 261-264 (1991)
17. Rossi GP, Seccia TM, Albertin G, Pessina AC. Measurement of endothelin: clinical and research use. Ann Clin Biochem 2000;37 (Pt 5):608-26
18. Shankar A, Loizidou M, Aliev G, Fredericks S, Holt D, Boulos PB, Burnstock G, Taylor I. Raised endothelin 1 levels in patients with colorectal liver metastases. Br J Surg 1998;85:502-6
19. Sokolovsky M, Endothelins and Sarafotoxins: Receptor Heterogeneity (Minireview); Int.J.Biochem. Vol.26, No.3, 335-340, 1994
20. Stewart DJ, Kubac G, Costello KB, Cernacek P. Increased plasma endothelin-1 in the early hours of acute myocardial infarction. J Am Coll Cardiol 1991;18:38-43
21. Stewart DJ, Levy RD, Cernacek P, Langleben D. Increased plasma endothelin-1 in pulmonary hypertension: marker or mediator of disease? Ann Intern Med 1991;114:464-9
22. Tschaikowsky K, Sagner S, Lehnert N, Kaul M, Ritter J. Endothelin in septic patients: effects on cardiovascular and renal function and its relationship to proinflammatory cytokines. Crit Care Med 2000;28:1854-60
23. Voerman HJ, Stehouwer CD, van Kamp GJ, Strack van Schijndel RJ, Groeneveld AB, Thijs LG. Plasma endothelin levels are increased during septic shock. Crit Care Med 1992;20:1097-101
24. Wanecek M, Weitzberg E, Rudehill A, Oldner A. The endothelin system in septic and endotoxin shock. Eur J Pharmacol 2000;407:1-15
25. Wei CM, Lerman A, Rodeheffer RJ, McGregor CG, Brandt RR, Wright S, Heublein DM, Kao PC, Edwards WD, Burnett JC, Jr. Endothelin in human congestive heart failure. Circulation 1994;89:1580-6
26. Weitzberg E, Lundberg JM, and Rudehill A, Elevated Plasma Levels of Endothelin in Patients With Sepsis Syndrome. Circulatory Shock 33:222-227 (1991)
27. Yanagisawa M, Kurihara H, Kimura S, Tomobe Y, Kobayashi M, Mitsui Y, Yazaki Y, Goto K, Masaki T. A novel potent vasoconstrictor peptide produced by vascular endothelial cells. Nature 1988;332:411-5

## Patentansprüche

1. In vitro-Verfahren zur Bestimmung der Bildung von Endothelinen bei Herz-Kreislauf-Erkrankungen, Entzündungen, Sepsis und Krebs, in Vollblut, Plasma oder Serum eines menschlichen Patienten zu Zwecken der medizinischen Diagnostik, **dadurch gekennzeichnet, dass** man die Bildung von Endothelin-1 (SEQ ID NO:2) und big-Endothelin-1 (SEQ ID NO:3) **dadurch** bestimmt, dass man solche C-terminalen Fragmente des Präpro-Endothelins-1 (SEQ ID NO:1) bestimmt, die von Antikörpern erkannt werden, die an Peptide binden, die Peptidsequenzen im Bereich der Aminosäuren 93 bis 212 des Präpro-Endothelins-1 entsprechen.

2. Verfahren nach Anspruch 1, **dadurch gekennzeichnet, dass** die Bestimmung in der biologischen Flüssigkeit mit Hilfe eines Immunoassays erfolgt, der mit mindestens einem markierten Antikörper, der spezifisch nur das zu bestimmende Peptidfragment erkennt, arbeitet.

3. Verfahren nach Anspruch 2, **dadurch gekennzeichnet, dass** der Immunoassay ein kompetitiver Immunoassay oder ein Sandwich-Immunoassay ist.

4. Verfahren nach Anspruch 1, **dadurch gekennzeichnet, dass** man solche C-terminalen Fragmente des Präpro-Endothelins-1 bestimmt, die von Antikörpern erkannt werden, die an Peptide binden, die Peptidsequenzen im Bereich der Aminosäuren 168 bis 212 (SEQ ID NO: 7) des Präpro-Endothelins-1 entsprechen.

5. Verfahren nach Anspruch 4, **dadurch gekennzeichnet, dass** man zur Bestimmung eines C-terminalen Fragments mit den Aminosäuren 168 bis 212 (SEQ ID NO:7) des Präpro-Endothelins-1 Paare von Antikörpern verwendet, die an zwei unterschiedliche Peptidsequenzen binden, die ausgewählt sind aus Peptidsequenzen mit den Aminosäuren 168-181, 184-203 und 200-212 des Präpro-Endothelins-1.

6. Verfahren nach einem der vorausgehenden Ansprüche, **dadurch gekennzeichnet, dass** es ein Verfahren zur quantitativen oder zur semiquantitativen Bestimmung der zu bestimmenden Peptidfragmente ist.

7. Verfahren nach Anspruch 6, **dadurch gekennzeichnet, dass** es ein immunchromatographischer Point-of-Care-Test oder ein anderer Schnelltest ist.

8. Verfahren nach einem der Ansprüche 4 bis 7, **dadurch gekennzeichnet, dass** die zur Bestimmung verwendeten Antikörper monoklonale und/oder affinitätsgereinigte polyklonale Antikörper sind.

9. Verfahren nach einem der Ansprüche 4 bis 8, **dadurch gekennzeichnet, dass** Antikörper verwendet werden, die durch Immunisierung eines Tiers mit einem Antigen erhalten wurden, das ein synthetisches Peptid enthält, das ausgewählt ist aus den Peptiden (SEQ ID NO:4), (SEQ ID NO:5) und (SEQ ID NO:6).

10. Verfahren nach einem der Ansprüche 4 bis 9, **dadurch gekennzeichnet, dass** zur Bestimmung zwei Antikörper verwendet werden, von denen einer markiert ist und der andere an eine Festphase gebunden ist oder selektiv an eine Festphase gebunden werden kann.

11. Verfahren nach einem der Ansprüche 4 bis 9, **dadurch gekennzeichnet, dass** zur Bestimmung zwei Antikörper verwendet werden, die beide in der flüssigen Reaktionsmischung dispergiert vorliegen, wobei an den ersten Antikörper eine erste Markierungskomponente gebunden ist, die Teil eines auf Fluoreszenz- oder Chemilumineszenz-Löschung oder -Verstärkung beruhenden Markierungssystems ist, und an den zweiten Antikörper die zweite Markierungskomponente dieses Markierungssystems gebunden ist, so dass nach Bindung beider Antikörper an das nachzuweisende Peptidfragment ein messbares Signal erzeugt wird, das eine Detektion der gebildeten Sandwichkomplexe in der Messlösung ermöglicht.

12. Verfahren nach Anspruch 11, **dadurch gekennzeichnet, dass** das Markierungssystem Seltenerdkrypate oder -chelate in Kombination mit einem Fluoreszenz- oder Chemilumineszenz-Farbstoff, insbesondere von Cyanintyp, umfaßt.

13. Verfahren nach einem der Ansprüche 1 bis 123, **dadurch gekennzeichnet, dass** es zur Diagnose, für die Bestimmung der Schweregrads und für die Prognostik sowie zur verlaufsbegleitenden Therapiekontrolle von Sepsis angewandt wird.

14. Verfahren nach Anspruch 13, **dadurch gekennzeichnet, dass** es im Rahmen einer Multiparameter-Bestimmung durchgeführt wird, bei der gleichzeitig mindestens ein weiterer für die Sepsisdiagnostik relevanter Parameter bestimmt wird.

15. Verfahren nach Anspruch 14, **dadurch gekennzeichnet, dass** der oder die weitere(n) für die Sepsisdiagnostik relevante(n) Parameter aus der Gruppe ausgewählt sind, die besteht aus Anti-Gangliosid-Antikörpern, den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (sCY1F), den Peptiden Inflammin und CHP, Fragmenten der Prohormone pro-ANP, pro-BNP oder pro-ADM, der Glycin-N-Acyltransferase (GNAT), der Carbamoylphosphat Synthetase 1 (CPS 1) und dem C-reaktiven Protein (CRP) oder Fragmenten davon.

16. Verfahren nach einem der Ansprüche 1 bis 12, **dadurch gekennzeichnet, dass** es auf dem Gebiet der Kardialdiagnostik angewandt wird.

17. Verfahren nach Anspruch 16, **dadurch gekennzeichnet, dass** es im Rahmen einer Multiparameter-Bestimmung durchgeführt wird, bei der gleichzeitig weitere für die Kardialdiagnostik relevante Parameter bestimmt werden.

18. Verfahren nach einem der Ansprüche 1 bis 12, **dadurch gekennzeichnet, dass** es auf dem Gebiet der Krebsdiagnostik angewandt wird.

19. Verfahren nach Anspruch 18, **dadurch gekennzeichnet, dass** es im Rahmen einer Multiparameter-Bestimmung durchgeführt wird, bei der gleichzeitig weitere für die Krebsdiagnostik relevante Parameter bestimmt werden.

20. Antikörper, die spezifisch an Peptide binden, die aus den Aminosäuresequenzen bestehen, die den Aminosäuren 168-181, 184-203 und 200-212 des Präpro-Endothelins-1 entsprechen.

21. Antikörper nach Anspruch 20, **dadurch gekennzeichnet, dass** sie affinitätsgereinigte polyklonale Antikörper oder monoklonale Antikörper sind.

22. Kit zur Durchfühunge eines Verfahrens nach einem der Ansprüche 1 bis 19, **dadurch gekennzeichnet, dass** er mindestens umfaßt: (a) einen ersten Antikörper gemäß einem der Ansprüche 20 und 21, (b) einen zweiten, anderen Antikörper gemäß einem der Ansprüche 20 und 21, wobei einer der Antikörper markiert ist und der andere immobilisiert oder immobiliserbar ist, sowie (c) ein Standardpeptid, das eine Aminosäuresequenz aufweist, die wenigstens die Aminosäuren 168-203 oder 168-212 des Präpro-Endothelins umfaßt.

23. Kit nach Anspruch 22, **dadurch gekennzeichnet, dass** der immobilisierte Antikörper an die Wände eines Teströhrchens immobilisiert vorliegt (CT).

## Claims

1. In vitro method for the determination of the formation of endothelins in cardiovascular diseases, inflammations, sepsis and cancer, in whole blood, plasma or serum of a human patient for purposes of medical diagnostics, **characterized in that** the formation of endothelin-1 (SEQ ID NO:2) and big endothelin-1 (SEQ ID NO:3) is determined by determining those C-terminal fragments of preproendothelin-1 (SEQ ID NO:1) which are recognized by antibodies which bind to peptides which correspond to peptide sequences in the range of amino acids 93 to 212 of preproendothelin-1.

2. Method according to Claim 1, **characterized in that** the determination in biological fluid is effected with the aid of an immunoassay which operates with at least one marked antibody which specifically recognizes only the peptide fragment to be determined.

3. Method according to Claim 2, **characterized in that** the immunoassay is a competitive immunoassay or a sandwich immunoassay.

4. Method according to claim 1, **characterized in that** those C-terminal fragments of preproendothelin-1 are determined which are recognized by antibodies which bind to peptides which correspond to peptide sequences in the range of the amino acids 168 to 212 (SEQ ID NO:7) of preproendothelin-1.

5. Method according to Claim 4, **characterized in that** pairs of antibodies which bind to two different peptide sequences which are selected from peptide sequences having the amino acids 168-181, 184-203 and 200-212 of preproendothelin-1 are used for determining a C-terminal fragment having amino acids 168 to 212 of preproendothelin-1 (SEQ ID NO:7).

6. Method according to any of the preceding Claims, which is a method for the quantitative or for the semiquantitative determination of the peptide fragments to be determined.

7. Method according to Claim 6, which is an immunochromatographic point-of-care test or another accelerated test.

8. Method according to any of Claims 4 to 7, **characterized in that** the antibodies used for the determination are monoclonal and/or affinity-purified polyclonal antibodies.

9. Method according to any of Claims 4 to 8, **characterized in that** antibodies which are obtained by immunizing an animal with an antigen which contains a synthetic peptide which is selected from the peptides (SEQ ID NO:4), (SEQ ID NO:5) and (SEQ ID NO:6) are used.

10. Method according to any of Claims 4 to 9, **characterized in that** two antibodies are used for the determination, one of which is marked and the other is bound to a solid phase or can be selectively bound to a solid phase.

11. Method according to any of Claims 4 to 9, **characterized in that** two antibodies are used for the determination, both of which are present in dispersed form in the liquid reaction mixture, a first marking component which is part of a marking system based on fluorescence or chemiluminescence distinction or amplification being bound to the first antibody, and the second marking component of this marking system being bound to the second antibody so that, after binding of both antibodies to the peptide fragment to be detected, a measurable signal which permits detection of the resulting sandwich complexes in the measuring solution is generated.

12. Method according to Claim 11, **characterized in that** the marking system comprises rare earth cryptates or chelates in combination with a fluorescent or chemiluminescent dye, in particular of the cyanine type.

13. Method according to any of Claims 1 to 12, which is used for diagnosis, for determination of severity and for prognosis and for monitoring the therapy in the course of sepsis.

14. Method according to Claim 13, which is carried out as part of a multiparameter determination, in which at least one further parameter relevant to sepsis diagnosis is determined simultaneously.

15. Method according to Claim 14, **characterized in that** the further parameter or parameters relevant for sepsis diagnosis is or are selected from the group which consists of anti-ganglioside antibodies, the proteins calcitonin, CA 125, CA 19-9, S100B, S100A proteins, LASP-1, soluble cytokeratin fragments, in particular CYFRA 21, TPS and/or soluble cytokeratin-1 fragments (sCY1F), the peptides inflammin and CHP, fragments of the prohormones pro-ANP, pro-BNP or pro-ADM, glycine-N-acyltransferase (GNAT), carbamoylphosphate synthetase 1 (CPS 1) and C-reactive protein (CRP) or fragments thereof.

16. Method according to any of Claims 1 to 12, which is used in the area of cardiac diagnostics.

17. Method according to Claim 16, which is carried out as part of a multiparameter determination, in which further parameters relevant to cardiac diagnostics are determined simultaneously.

18. Method according to any of Claims 1 to 12, which is used in the area of cancer diagnostics.

19. Method according to Claim 18, which is carried out as part of a multiparameter determination, in which further parameters relevant to cancer diagnostics are determined simultaneously.

20. Antibodies which bind specifically to peptides which consist of the amino acid sequences which correspond to the amino acids 168-181, 184-203 and 200-212 of preproendothelin-1.

21. Antibodies according to Claim 20, which are affinity-purified polyclonal antibodies or monoclonal antibodies.

22. Kit for carrying out a method according to any of Claims 1 to 19, which comprises at least: (a) a first antibody as claimed in either of claims 20 and 21, (b) a second, different antibody as claimed in either of claims 20 and 21, one of the antibodies being marked and the other being immobilized or immobilizable, and (c) a standard peptide which has an amino acid sequence which comprises at least the amino acids 168-2.03 or 168-212 of preproendothelin.

23. Kit according to Claim 22, **characterized in that** the immobilized antibody is present in immobilized form on the walls of a test tube (CT).

## Revendications

1. Procédé *in-vitro* pour déterminer la formation d'endothélines en vue du diagnostic médical de maladies cardio-vasculaires, d'inflammations, de septicémies et de cancers, dans le sang, le plasma ou le sérum d'un patient humain, **caractérisé en ce que** l'on détermine la formation d'endothéline-1 (SEQ ID NO:2) et de big-endothéline 1 (SEQ ID NO:3) en déterminant les fragments C-terminaux de la prépro-endothéline-1 (SEQ ID NO:1), qui sont reconnus par les anticorps qui se lient à des peptides qui correspondent aux séquences de peptides de la région des acides aminés 93 à 212 de la prépro-endothéline-1.

2. Procédé selon la revendication 1, **caractérisé en ce que** la détermination est réalisée dans le liquide biologique au moyen d'un test immunologique qui travaille avec au moins un anticorps marqué qui reconnaît spécifiquement et uniquement le fragment de peptide à déterminer.

3. Procédé selon la revendication 2, **caractérisé en ce que** le test immunologique est un test immunologique par compétition ou un test immunologique en sandwich.

4. Procédé selon la revendication 1, **caractérisé en ce que** l'on détermine les fragments C-terminaux de la prépro-endothéline-1 (SEQ ID NO:1) qui sont reconnus par les anticorps qui se lient à des peptides qui correspondent aux séquences de peptides dans la région des acides aminés 168 à 212 de la prépro-endothéline-1 (SEQ ID NO:7).

5. Procédé selon la revendication 4, **caractérisé en ce que**, pour déterminer un fragment C-terminal qui présente les acides aminés 168 à 212 (SEQ ID NO:7) de la prépro-endothéline-1, on utilise des paires d'anticorps qui se lient à deux séquences différentes de peptides qui sont sélectionnées parmi les séquences de peptides qui présentent les acides aminés 168 à 181, 184 à 203 et 200 à 212 de la prépro-endothéline-1.

6. Procédé selon l'une des revendications précédentes, **caractérisé en ce qu'**il s'agit d'un procédé de détermination quantitative ou semi-quantitative des fragments de peptide à déterminer.

7. Procédé selon la revendication 6, **caractérisé en ce qu'**il s'agit d'un test immunochromatographique réalisé sur le lieu des soins ou d'un autre test rapide.

8. Procédé selon l'une des revendications 4 à 7, **caractérisé en ce que** les anticorps utilisés pour la détermination sont des anticorps monoclonaux et/ou des anticorps polyclonaux purifiés par affinité.

9. Procédé selon l'une des revendications 4 à 8, **caractérisé en ce que** l'on utilise des anticorps obtenus par immunisation d'un animal au moyen d'un antigène qui contient un peptide de synthèse sélectionné parmi les peptides (SEQ ID NO:4), (SEQ ID NO:5) et (SEQ ID NO:6).

10. Procédé selon l'une des revendications 4 à 9, **caractérisé en ce que**, pour la détermination, on utilise deux anticorps dont l'un est marqué et l'autre est lié à une phase solide ou peut être lié sélectivement à une phase solide.

11. Procédé selon l'une des revendications 4 à 9, **caractérisé en ce que**, pour la détermination, on utilise deux anticorps qui sont tous les deux en dispersion dans le mélange liquide de réaction, un premier composant de marquage qui fait partie d'un système de marquage basé sur l'extinction ou le renforcement de la fluorescence ou de la chimiluminescence étant lié au premier anticorps et le deuxième composant de marquage de ce système de marquage étant lié au deuxième anticorps, de telle sorte qu'après la liaison des deux anticorps au fragment de peptide à détecter, un signal mesurable est généré et permet de détecter le complexe sandwich formé dans la solution de mesure.

12. Procédé selon la revendication 11, **caractérisé en ce que** le système de marquage comprend des cryptates ou chélates de terres rares en combinaison avec un colorant de fluorescence ou de chimiluminescence et en particulier du type de la cyanine.

13. Procédé selon l'une des revendications 1 à 12, **caractérisé en ce qu'**on l'utilise pour le diagnostic, pour déterminer le degré de gravité et pour le pronostic ainsi que pour le contrôle d'une thérapie qui accompagne l'évolution de la septicémie.

14. Procédé selon la revendication 13, **caractérisé en ce qu'**on l'exécute dans le cadre d'une détermination de plusieurs paramètres, lors de laquelle on détermine en même temps d'autres paramètres pertinents pour le diagnostic de la septicémie.

15. Procédé selon la revendication 14, **caractérisé en ce que** le ou les autres paramètres pertinents pour le diagnostic de la septicémie sont sélectionnés dans le groupe constitué des anticorps antigangliosides, des protéines procalcitonine, CA 125, CA 19-9, S100B, S100A, LASP-1, des fragments solubles de la cytokératine et en particulier CYFRA 21, TPS et/ou les fragments solubles de la cytokératine-1 (sCY1F), les peptides inflammine et CHP, les fragments des prohormones pro-ANP, pro-BNP ou pro-ADM, de la glycine-N-acyle transférase (GNAT), de la carbamoylphosphate synthétase 1 (CPS 1) et de la protéine C-réactive (CRP), ou des fragments de ces éléments.

16. Procédé selon l'une des revendications 1 à 12, **caractérisé en ce qu'**il est utilisé dans le domaine du diagnostic cardiaque.

17. Procédé selon la revendication 16, **caractérisé en ce qu'**on l'exécute dans le cadre d'une détermination de plusieurs paramètres, lors de laquelle on détermine en même temps d'autres paramètres pertinents pour le diagnostic cardiaque.

18. Procédé selon l'une des revendications 1 à 12, **caractérisé en ce qu'**il est utilisé dans le domaine du diagnostic de cancer.

19. Procédé selon la revendication 18, **caractérisé en ce qu'**on l'exécute dans le cadre d'une détermination de plusieurs paramètres, lors de laquelle on détermine en même temps d'autres paramètres pertinents pour le diagnostic de cancer.

20. Anticorps qui se lient spécifiquement aux peptides constitués des séquences d'acides aminés qui correspondent aux acides aminés 168 à 181, 184 à 203 et 200 à 212 de la prépro-endothéline-1.

21. Anticorps selon la revendication 20, **caractérisé en ce qu'**il s'agit d'anticorps polyclonaux purifiés par affinité ou d'anticorps monoclonaux.

22. Trousse pour exécuter un procédé selon l'une des revendications 1 à 19, **caractérisée en ce qu'**elle contient au moins (a) un premier anticorps selon l'une des revendications 20 et 21, (b) un deuxième et autre anticorps selon l'une des revendications 20 et 21, l'un des anticorps étant marqué et l'autre étant immobilisé ou apte à être immobilisé et (c) un peptide étalon qui présente une séquence d'acides aminés qui comprend au moins les acides aminés 168 à 203 ou 168 à 212 de la prépro-endothéline.

23. Trousse selon la revendication 22, **caractérisée en ce que** l'anticorps immobilisé est immobilisé sur les parois d'un petit tube à essai (CT).


### SEQUENCE LISTING

<110> B.R.A.H.M.S Aktiengesellschaft
<120> In vitro-Verfahren zur Bestimmung der Bildung von Endothelinen zu diagnostischen Zwecken
<130> 4295EP
<140>
   <141>
<160> 7
<170> PatentIn Ver. 2.1
<210> 1
   <211> 212
   <212> PRT
   <213> Homo sapiens
<400> 1
   Met Asp Tyr Leu Leu Met Ile Phe Ser Leu Leu Phe Val Ala Cys Gln
   1               5                   10                  15
   Gly Ala Pro Glu Thr Ala Val Leu Gly Ala Glu Leu Ser Ala Val Gly
               20                  25                  30
   Glu Asn Gly Gly Glu Lys Pro Thr Pro Ser Pro Pro Trp Arg Leu Arg
           35                  40                  45
   Arg Ser Lys Arg Cys Ser Cys Ser Ser Leu Met Asp Lys Glu Cys Val
       50                  55                  60
   Tyr Phe Cys His Leu Asp Ile Ile Trp Val Asn Thr Pro Glu His Val
   65                  70                  75                  80
   Val Pro Tyr Gly Leu Gly Ser Pro Arg Ser Lys Arg Ala Leu Glu Asn
                   85                  90                  95
   Leu Leu Pro Thr Lys Ala Thr Asp Arg Glu Asn Arg Cys Gln Cys Ala
               100                 105                 110
   Ser Gln Lys Asp Lys Lys Cys Trp Asn Phe Cys Gln Ala Gly Lys Glu
           115                 120                 125
   Leu Arg Ala Glu Asp Ile Met Glu Lys Asp Trp Asn Asn His Lys Lys
       130                 135                 140
   Gly Lys Asp Cys Ser Lys Leu Gly Lys Lys Cys Ile Tyr Gln Gln Leu
   145                 150                 155                 160
   Val Arg Gly Arg Lys Ile Arg Arg Ser Ser Glu Glu His Leu Arg Gln
                   165                 170                 175
   Thr Arg Ser Glu Thr Met Arg Asn Ser Val Lys Ser Ser Phe His Asp
               180                 185                 190
   Pro Lys Leu Lys Gly Lys Pro Ser Arg Glu Arg Tyr Val Thr His Asn
           195                 200                 205
   Arg Ala His Trp
       210
<210> 2
   <211> 21
   <212> PRT
   <213> Homo sapiens
<400> 2
   Cys Ser Cys Ser Ser Leu Met Asp Lys Glu Cys Val Tyr Phe Cys His
   1               5                   10                  15
   Leu Asp Ile Ile Trp
               20
<210> 3
   <211> 38
   <212> PRT
   <213> Homo sapiens
<400> 3
   Cys Ser Cys Ser Ser Leu Met Asp Lys Glu Cys Val Tyr Phe Cys His
   1 5 10 15
   Leu Asp Ile Ile Trp Val Asn Thr Pro Glu His Val Val Pro Tyr Gly
   20 25 30
   Leu Gly Ser Pro Arg Ser
   35
<210> 4
   <211> 15
   <212> PRT
   <213> Artificial Sequence
<220>
   <223> Description of Artificial Sequence: Partial EN1 Sequence with terminal C
<400> 4
   Cys Arg Ser Ser Glu Glu His Leu Arg Gln Thr Arg Ser Glu Thr
   1 5 10 15
<210> 5
   <211> 14
   <212> PRT
   <213> Artificial Sequence
<220>
   <223> Description of Artificial Sequence: Partial EN1 sequence with terminal C
<400> 5
   Cys Ser Arg Glu Arg Tyr Val Thr His Asn Arg Ala His Trp
   1 5 10
<210> 6
   <211> 20
   <212> PRT
   <213> Artificial Sequence
<220>
   <223> Description of Artificial Sequence: Partial EN1 sequence with terminal C
<400> 6
   Asn Ser Val Lys Ser Ser Phe His Asp Pro Lys Leu Lys Gly Lys Pro
   1 5 10 15
   Ser Arg Glu Arg
   20
<210> 7
   <211> 44
   <212> PRT
   <213> Homo sapiens
<400> 7
   Ser Ser Glu Glu His Leu Arg Gln Thr Arg Ser Glu Thr Met Arg Asn
    1               5                  10                  15
   Ser Val Lys Ser Ser Phe His Asp Pro Lys Leu Lys Gly Lys Pro Ser
               20                  25                  30
   Arg Glu Arg Tyr Val Thr His Asn Arg Ala His Trp
           35                  40